# EUROPEAN PATENT SPECIFICATION

(11) **EP 4 640 155 B1**
(45) Date of publication and mention of the grant of the patent: **14.10.2026**
(21) Application number: 24775188.6
(22) Date of filing: 20.03.2024
(51) Int. Cl.: A61B 6/00, A61B 6/03, A61B 6/51, A61B 5/00, G06T 7/30, G06V 10/75, G06V 10/80, G06V 10/82, G06V 20/64, G06V 20/70, G06V 40/16

(54) **METHOD FOR GENERATING DENTAL ALIGNMENT DATA**
VERFAHREN ZUR ERZEUGUNG VON DENTALEN AUSRICHTUNGSDATEN
PROCÉDÉ DE GÉNÉRATION DE DONNÉES D'ALIGNEMENT DENTAIRE

(30) Priority: 21.03.2023 KR 20230036712; 13.03.2024 KR 20240035108
(43) Date of publication of application: 29.10.2025
(73) Proprietor: Ray Co., Ltd., Seongnam-si, Gyeonggi-do 13494 (KR)
(72) Inventor: SHIN, Seung Hoon, Seongnam-si Gyeonggi-do 13494 (KR); SEONG, Hyun Chan, Seongnam-si Gyeonggi-do 13494 (KR); CHAE, Yun Hyeon, Seongnam-si Gyeonggi-do 13309 (KR); KIM, Yeong Mi, Seongnam-si Gyeonggi-do 13535 (KR); MIN, Gyeong Uk, Seongnam-si Gyeonggi-do 13494 (KR); PARK, Seok Jun, Suwon-si Gyeonggi-do 16509 (KR)
(74) Representative: Studio Torta S.p.A.
(86) International application number: PCT/KR2024/003479
(87) International publication number: WO 2024/196140

(56) References cited:
- JP-A- 2017 200 537
- KR-A- 20130 075 305
- KR-A- 20180 102 725
- KR-A- 20230 022 614
- US-A1- 2020 175 678
- US-A1- 2022 068 039
- US-A1- 2022 361 992
- US-B2- 10 952 814

## Description

### Technical Field

The present disclosure relates to a method for generating dental registration data, and more particularly, to a method for generating dental registration data, in which an intraoral scan image and a CT scan image are registered, to more intuitively show a dental patient's tooth condition.

### Background Art

In dental consultation or treatment, there is a limit to relying solely on the verbal explanations of a dentist, so there is a need to show the patient the current state of their teeth and the state after treatment more intuitively.

Accordingly, with the development of digital imaging technology, various types of image data are being widely used in dental treatment or consultation. The use of such various image data is enabling improved dental diagnosis, treatment planning, patient management, and the like. Representative image data used in dental consultation or treatment may include a CT (Computerized Tomography) scan image obtained by capturing images with dental CT equipment, a face image obtained by capturing a patient's face with a face capturing device, and an intraoral scan image obtained by scanning the inside of a patient's oral cavity using a 3D intraoral scanner.

However, in the case of CT scanning, in order to fix the patient's position, the patient bites on a specific part of the CT scanning device (e.g., a mouthpiece, a bite block) while the image is being captured. The CT scan image obtained in this way is an image taken with the oral cavity slightly open, and may differ from the actual oral occlusion state during normal times. Therefore, it was cumbersome for a user such as a dentist to appropriately load and utilize image data having different uses according to the purpose of treatment or consultation.

For example, when determining an implant placement position and performing a surgical guide design for an implant procedure, it is important to accurately check the position of the nerve canal, so an intraoral scan image registered (aligned) with a CT scan image in a CT occlusion state may be necessary. Meanwhile, when planning prosthetic treatment such as a crown, it is necessary to check the part where the tooth to be treated and the opposing tooth meet, so an intraoral scan image registered with a CT scan image in an intraoral scan occlusion state may be necessary. Relevant prior art regarding the registration of intraoral and CT dental scan images is taught in US 2022/068039 A1, US 2022/361992 A1 and US 2020/175678 A1.

Accordingly, there is a demand for a method of generating and displaying appropriate registration data according to a desired type of treatment, regardless of the purpose of dental treatment or consultation.

### DISCLOSURE OF INVENTION

### Technical Problem

Accordingly, an object of the present disclosure is to provide a method for generating and displaying appropriate registration data according to a desired type of treatment, regardless of the purpose of dental treatment or consultation.

According to an embodiment of the present disclosure, in registering (or aligning) a maxillary scan image and a mandibular scan image with a CT scan image, the mandibular scan image may be registered based on the maxillary scan image registered with the CT scan image. In this case, a set of intraoral scan images aligned with the CT in an intraoral scan occlusion state may be obtained.

According to an embodiment of the present disclosure, in registering (or aligning) a maxillary scan image and a mandibular scan image with a CT scan image, the maxillary scan image registered with the CT scan image and the mandibular scan image registered with the CT scan image may be registered with each other. In this case, a set of intraoral scan images aligned with the CT in a CT occlusion state may be obtained.

According to an embodiment of the present disclosure, a technical object is to provide at least one of registration data representing an intraoral scan occlusion state and registration data representing a CT occlusion state according to the purpose of treatment.

However, the problems to be solved by the present disclosure are not limited to those mentioned above, and may include other purposes that can be clearly understood by those skilled in the art from the following description, although not mentioned.

### Technical Solution to Problem

Hereinafter, specific means for achieving the objects of the present disclosure will be described.

A method for generating dental registration data according to an embodiment of the present disclosure may include: a step of acquiring an intraoral scan image of a patient's dental region - the intraoral scan image including a maxillary scan image and a mandibular scan image -; a step of acquiring a CT scan image including the patient's dental region; a step of generating first sub-registration data by registering the maxillary scan image with the CT scan image; a step of generating first registration data by registering the mandibular scan image with the first sub-registration data; a step of generating second sub-registration data by registering the mandibular scan image with the CT scan image; and a step of generating second registration data by registering the first sub-registration data with the second sub-registration data.

The aforementioned method for generating dental registration data may further include: a step of receiving a user input for selecting one of the first registration data and the second registration data; and a step of displaying the selected one registration data in response to receiving the user input.

The aforementioned method for generating dental registration data may further include: a step of receiving a user input regarding a treatment method for the patient's dental region; a step of selecting one of the first registration data and the second registration data corresponding to the user input; and a step of displaying the selected one registration data.

The aforementioned method for generating dental registration data may further include a step of obtaining a CT segmentation image in which at least one of maxillary teeth, mandibular teeth, a maxillary bone, a mandibular bone, and a mandibular nerve canal is segmented by performing image processing on the CT scan image.

In the aforementioned method for generating dental registration data, the step of generating the first sub-registration data may include a step of generating the first sub-registration data by registering the maxillary scan image with the CT segmentation image, and the step of generating the second sub-registration data may include a step of generating the second sub-registration data by registering the mandibular scan image with the CT segmentation image.

In the aforementioned method for generating dental registration data, the step of generating the first registration data may include: a step of calculating a transformation matrix between the first registration data and the second sub-registration data; and a step of applying an inverse transformation matrix for the transformation matrix to at least one of the segmented mandibular teeth, mandibular bone, and mandibular nerve canal.

The aforementioned method for generating dental registration data may further include: a step of acquiring a 3-dimensional face image for a face including the patient's dental region; and a step of generating a dental 3D avatar as final registration data by registering the 3-dimensional face image with one of the CT scan image, the first registration data, and the second registration data.

In the aforementioned method for generating dental registration data, the step of generating the first sub-registration data may include a step of generating the first sub-registration data from the maxillary scan image and the CT scan image using a first deep learning model, and the first deep learning model may be an artificial intelligence model constructed by modeling a correlation between input data and output data according to a deep learning algorithm, where the input data is a plurality of maxillary scan images and a plurality of CT scan image sets for a plurality of patients, and the output data is a plurality of sub-registration data sets in which each of the plurality of maxillary scan images is registered with each of the plurality of CT scan images.

In the aforementioned method for generating dental registration data, the step of generating the second sub-registration data may include a step of generating the second sub-registration data from the mandibular scan image and the CT scan image using a second deep learning model, and the second deep learning model may be an artificial intelligence model constructed by modeling a correlation between input data and output data according to a deep learning algorithm, where the input data is a plurality of mandibular scan images and a plurality of CT scan image sets for a plurality of patients, and the output data is a plurality of sub-registration data sets in which each of the plurality of mandibular scan images is registered with each of the plurality of CT scan images.

In the aforementioned method for generating dental registration data, the first sub-registration data and the second sub-registration data may be generated based on at least three landmarks of the patient's 3-dimensional data, and the at least three landmarks may be extracted by an application for generating dental registration data, wherein the at least three landmarks are extracted using an artificial neural network module embedded in the application or connected via a network, and the artificial neural network module may be pre-trained through pre-training data.

A dental registration data generation device according to an embodiment of the present disclosure may include a communication circuit, a memory, and a processor, and the processor may be configured to acquire an intraoral scan image of a patient's dental region - the intraoral scan image including a maxillary scan image and a mandibular scan image -, acquire a CT scan image including the patient's dental region, generate first sub-registration data by registering the maxillary scan image with the CT scan image, generate first registration data by registering the mandibular scan image with the first sub-registration data, generate second sub-registration data by registering the mandibular scan image with the CT scan image, and generate second registration data by registering the first sub-registration data with the second sub-registration data.

In the aforementioned dental registration data generation device, the device may further include an input device and a display, and the processor may be configured to receive a user input for selecting one registration data from the first registration data and the second registration data through the input device, and display the selected one registration data through the display in response to receiving the user input.

In the aforementioned dental registration data generation device, the device may further include an input device and a display, and the processor may be configured to receive a user input regarding a treatment method for the patient's dental region through the input device, select one registration data corresponding to the user input from the first registration data and the second registration data, and display the selected one registration data through the display.

In the aforementioned dental registration data generation device, the processor may be configured to obtain a CT segmentation image in which at least one of maxillary teeth, mandibular teeth, a maxillary bone, a mandibular bone, and a mandibular nerve canal is segmented by performing image processing on the CT scan image.

In the aforementioned dental registration data generation device, the processor may be configured to generate the first sub-registration data by registering the maxillary scan image with the CT segmentation image, and generate the second sub-registration data by registering the mandibular scan image with the CT segmentation image.

In the aforementioned dental registration data generation device, the processor may, in the step of generating the first registration data, calculate a transformation matrix from the first registration data to the second sub-registration data, and apply an inverse transformation matrix for the transformation matrix to at least one of the segmented mandibular teeth, mandibular bone, and mandibular nerve canal.

In the aforementioned dental registration data generation device, the processor may be configured to acquire a 3-dimensional face image for a face including the patient's dental region, and generate a dental 3D avatar as final registration data by registering the 3-dimensional face image with one of the CT scan image, the first registration data, and the second registration data.

In the aforementioned dental registration data generation device, the processor may be configured to generate the first sub-registration data from the maxillary scan image and the CT scan image using a first deep learning model, and the first deep learning model may be an artificial intelligence model constructed by modeling a correlation between input data and output data according to a deep learning algorithm, where the input data is a plurality of maxillary scan images and a plurality of CT scan image sets for a plurality of patients, and the output data is a plurality of sub-registration data sets in which each of the plurality of maxillary scan images is registered with each of the plurality of CT scan images.

In the aforementioned dental registration data generation device, the processor may be configured to generate the second sub-registration data from the mandibular scan image and the CT scan image using a second deep learning model, and the second deep learning model may be an artificial intelligence model constructed by modeling a correlation between input data and output data according to a deep learning algorithm, where the input data is a plurality of mandibular scan images and a plurality of CT scan image sets for a plurality of patients, and the output data is a plurality of sub-registration data sets in which each of the plurality of mandibular scan images is registered with each of the plurality of CT scan images.

In the aforementioned dental registration data generation device, the first sub-registration data and the second sub-registration data may be generated based on at least three landmarks of the patient's 3-dimensional data, and the at least three landmarks may be extracted by an application for generating dental registration data, wherein the at least three landmarks are extracted using an artificial neural network module embedded in the application or connected via a network, and the artificial neural network module may be pre-trained through pre-training data.

### Advantageous Effects of Invention

According to an embodiment of the present disclosure, at least one of registration data representing an intraoral scan occlusion state and registration data representing a CT occlusion state may be provided according to the purpose of treatment.

However, the effects that can be obtained from the present disclosure are not limited to the effects mentioned above, and other unmentioned effects will be clearly understood by those skilled in the art to which the present disclosure pertains from the description below.

### BRIEF DESCRIPTION OF DRAWINGS

The following drawings attached to this specification illustrate embodiments of the present disclosure, and further describe aspects and features of the present disclosure together with the detailed description of the present disclosure. Thus, the present disclosure should not be construed as being limited to the drawings.
FIG. 1 is a diagram for schematically explaining a basic configuration of a dental registration data generation device according to an embodiment of the present disclosure.
FIG. 2 is a block diagram of a dental registration data generation device according to an embodiment of the present disclosure.
FIG. 3 is a diagram for schematically explaining the principle of generating dental registration data by a registration module according to an embodiment of the present disclosure.
FIG. 4 is an operational flowchart for explaining a method for generating dental registration data according to an embodiment of the present disclosure.
FIG. 5A is a CT scan image according to an embodiment of the present disclosure. FIG. 5B is a maxillary scan image among intraoral scan images according to an embodiment of the present disclosure. FIG. 5C is first sub-registration data according to an embodiment of the present disclosure.
FIG. 6A is a mandibular scan image among intraoral scan images according to an embodiment of the present disclosure. FIG. 6B is first registration data according to an embodiment of the present disclosure.
FIG. 7 is second sub-registration data according to an embodiment of the present disclosure.
FIG. 8 is second registration data according to an embodiment of the present disclosure.
FIG. 9 is an operational flowchart for explaining a method for generating dental registration data according to an embodiment of the present disclosure.
FIGS. 10A and 10B are CT segmentation images according to an embodiment of the present disclosure.
FIG. 11 is first registration data according to an embodiment of the present disclosure.
FIG. 12 is second registration data according to an embodiment of the present disclosure.

### DESCRIPTION OF EMBODIMENTS

Various embodiments described in this specification are illustrated for the purpose of clearly explaining the technical idea of the present disclosure, and are not intended to limit the technical idea to a specific embodiment. The technical idea of the present disclosure includes various modifications, equivalents, alternatives, and embodiments selectively combined from all or part of each embodiment described in this specification. In addition, the scope of rights of the technical idea of the present disclosure is not limited to the various embodiments presented below or the detailed descriptions thereof.

Unless otherwise defined, terms used in this specification, including technical or scientific terms, may have meanings that are generally understood by a person of ordinary skill in the art to which the present disclosure pertains.

Expressions such as "comprises," "may comprise," "has," "may have," "includes," and "may include" used in this specification mean that the feature in question (e.g., function, operation, or component) exists, and do not exclude the existence of other additional features. That is, such expressions should be understood as open-ended terms implying the possibility of including a second embodiment.

In this specification, singular expressions include plural expressions unless the context clearly indicates otherwise. In addition, plural expressions include singular expressions unless the context clearly indicates otherwise. Throughout the specification, when a part is said to include a certain component, it means that it may further include other components, not excluding other components, unless there is a specific statement to the contrary.

Also, the term 'module' or 'unit' as used in the specification refers to a software or hardware component, and a 'module' or 'unit' performs certain functions. However, the meaning of a 'module' or 'unit' is not limited to software or hardware. A 'module' or 'unit' may be configured to reside in an addressable storage medium and may be configured to execute one or more processors. Thus, as an example, a 'module' or 'unit' may include at least one of components such as software components, object-oriented software components, class components, and task components, and processes, functions, attributes, procedures, subroutines, segments of program code, drivers, firmware, microcode, circuitry, data, databases, data structures, tables, arrays, or variables. The functions provided within the components and 'modules' or 'units' may be combined into a smaller number of components and 'modules' or 'units', or may be further divided into additional components and 'modules' or 'units'.

According to an embodiment of the present disclosure, a 'module' or 'unit' may be implemented with a processor and memory. A 'processor' should be broadly interpreted to include a general-purpose processor, a central processing unit (CPU), a microprocessor, a digital signal processor (DSP), a controller, a microcontroller, a state machine, and the like. In some contexts, a 'processor' may also refer to an application-specific integrated circuit (ASIC), a programmable logic device (PLD), a field-programmable gate array (FPGA), etc. A 'processor' may also refer to a combination of processing devices, such as, for example, a combination of a DSP and a microprocessor, a combination of a plurality of microprocessors, a combination of one or more microprocessors coupled with a DSP core, or any other such configuration. Furthermore, 'memory' should be broadly interpreted to include any electronic component capable of storing electronic information. 'Memory' may refer to various types of processor-readable media such as random-access memory (RAM), read-only memory (ROM), non-volatile random-access memory (NVRAM), programmable read-only memory (PROM), erasable programmable read-only memory (EPROM), electrically erasable PROM (EEPROM), flash memory, magnetic or optical data storage, registers, and so on. Memory is said to be in electronic communication with a processor if the processor can read information from and/or write information to the memory. Memory that is integrated into a processor is in electronic communication with the processor.

Expressions such as "first," "second," or "first," "second" used in this specification are used to distinguish one object from another among a plurality of objects of the same kind, unless the context means otherwise, and do not limit the order or importance between the objects.

Expressions used herein, such as "A, B, and C," "A, B, or C," "A, B, and/or C," or "at least one of A, B, and C," "at least one of A, B, or C," "at least one of A, B, and/or C," "at least one selected from A, B, and C," "at least one selected from A, B, or C," "at least one selected from A, B, and/or C," and the like, may mean each respective listed item or all possible combinations of the listed items. For example, "at least one selected from A and B" may refer to all of (1) A, (2) at least one of A, (3) B, (4) at least one of B, (5) at least one of A and at least one of B, (6) at least one of A and B, (7) at least one of B and A, and (8) both A and B.

The expression "based on" used in this specification is used to describe one or more factors that influence the act or operation of determination or judgment described in the phrase or sentence containing the expression, and this expression does not exclude additional factors that influence the act or operation of the determination or judgment.

As used herein, the expression that a component (e.g., a first component) is "connected to" or "coupled to" another component (e.g., a second component) may mean not only that the component is directly connected or coupled to the other component, but also that it is connected or coupled through a new, different component (e.g., a third component).

The expression "configured to," as used herein, may, depending on the context, have meanings such as "set to," "having the capability to," "modified to," "made to," "able to," and the like. This expression is not limited to the meaning of "specifically designed in hardware." For example, a processor "configured to" perform a specific operation may mean a general-purpose processor that can perform that specific operation by executing software.

Hereinafter, various embodiments of the present disclosure will be described with reference to the accompanying drawings. In the accompanying drawings and the description of the drawings, the same or substantially equivalent components may be given the same reference numerals. In addition, in the following description of various embodiments, redundant description of the same or corresponding components may be omitted, but this does not mean that the corresponding component is not included in the embodiment.

### [Basic Structure of Dental Registration Data Generation Device]

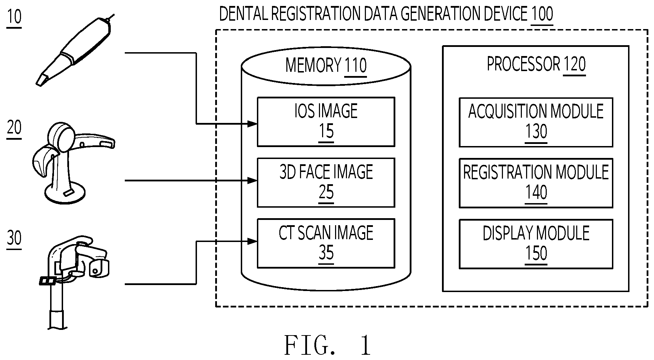

FIG. 1 is a diagram for schematically explaining a basic configuration of a dental registration data generation device according to an embodiment of the present disclosure.

Referring to FIG. 1, a dental registration data generation device 100 may include a memory 110 and a processor 120.

The memory 110 may store personal information and treatment data of each patient, various applications including an application for generating dental registration data, and the like. According to an embodiment, the patient's treatment data may include at least one of an Intra Oral Scan (IOS) image 15, a 3-dimensional (3D) face image 25, and a Computed Tomography (CT) scan image 35.

The processor 120 may execute an application for generating dental registration data stored in the memory 110. According to an embodiment, an acquisition module 130, a registration module 140, and a display module 150 may be implemented as software upon execution of the application for generating dental registration data. However, depending on the design of the device, some modules may be implemented as hardware, and this is not limited thereto.

According to an embodiment, an Intra Oral Scan (IOS) image 15 may be obtained by scanning a patient's dental region, that is, the inside of the oral cavity, using a 3-dimensional intraoral scanner 10. The 3-dimensional intraoral scanner 10 is a non-contact scanner that may collect surface data of the structure inside the patient's oral cavity and generate a 3D Intra Oral Scan (IOS) image based on the surface data using a confocal method or an optical triangulation method. When a 3D Intra Oral Scan (IOS) image is obtained as a digital impression through the 3-dimensional intraoral scanner 10 in this way, there is an advantage that there is no need to go through a cumbersome procedure of physically obtaining the structure inside the patient's oral cavity by performing an impression-taking operation (molding operation) using an impression material contained in a tray. The Intra Oral Scan (IOS) image 15 may be obtained through various types of 3-dimensional intraoral scanners available on the market, and is not limited thereto.

The intraoral scan image may include a maxillary scan image and a mandibular scan image. The maxillary scan image may be obtained by scanning the patient's maxillary teeth, and the mandibular scan image may be obtained by scanning the patient's mandibular teeth.

A 3D face image 25 may be obtained by photographing a patient's face using a 3-dimensional face capturing device 20. According to an embodiment, the 3-dimensional face capturing device 20 may include a plurality of cameras, and may be operated in a manner in which the plurality of cameras capture the patient's face in a single shot to obtain the 3-dimensional face image 25. However, the 3-dimensional face capturing device 20 may also be operated in a manner of capturing the patient's face at different times while the patient's face is fixed or moving, and is not limited thereto.

In addition, according to an embodiment, the plurality of cameras of the 3-dimensional face capturing device 20 may include at least one dental capturing camera for capturing the patient's dental region, along with a plurality of face capturing cameras. The plurality of face capturing cameras may be installed on the front, left, right, and bottom of the 3-dimensional face capturing device 20 so as to be able to capture the front, left, right, and bottom of the patient's face. According to an embodiment, the dental capturing camera may be different from the face capturing camera, for example, the magnification of the lens may be different. The dental capturing camera may, for example, be installed on the left and right sides of the 3-dimensional face capturing device 20 and configured to be able to capture the left and right sides of the patient's dental region, but the number or installation position of the dental capturing cameras is not limited thereto. The provision of a separate dental capturing camera in this way is not only for presenting the patient's teeth more accurately, but also has the aspect of enabling more precise and smooth registration with other data (for example, a CT scan image or an Intra Oral Scan (IOS) image) in the generation of registration data. This will be described in detail later.

According to an embodiment, the 3D face image 25 may be captured with the patient's dental region exposed. That is, when capturing the patient's face with the 3-dimensional face capturing device 20, the patient may be guided to fix his or her face in a proper position and expose the dental region, and then the capturing may be performed. To this end, the 3-dimensional face capturing device 20 may be designed to have a guide function. Accordingly, not only the patient's face but also the dental region may be captured more accurately to generate the 3D face image 25. However, in some cases, it may not be necessary to obtain the 3D face image 25 with the patient's dental region exposed, and in such a case, the 3D face image 25 may or may not show the teeth.

According to an embodiment, the 3-dimensional face capturing device 20 may obtain the 3D face image 25 based on a plurality of images obtained by capturing the patient's face using a plurality of cameras while irradiating a structured light pattern onto the patient's face. However, the method of obtaining the 3D face image 25 may include various methods such as a laser scanning method, a method using a depth sensor, and a method of reconstructing a 3D face image using artificial intelligence (AI), in addition to the method using structured light, and is not limited thereto.

The CT scan image 35 may be obtained by capturing a face area including the patient's dental region through a CT device 30. The CT apparatus (30) is a dental CT device, which may be, for example, a Cone Beam CT (CBCT) apparatus. A cone-beam CT apparatus offers the advantage of acquiring CT images of a patient's dental region with minimal radiation exposure by projecting a cone-shaped X-ray beam. However, the CT apparatus (30) may also be a fan-beam CT apparatus, which is a device that projects a fan-shaped X-ray beam. That is, the CT scan image 35 according to the embodiments of the present disclosure may be obtained through various CT devices, and is not limited thereto.

According to an embodiment, the acquisition module 130 of the dental registration data generation device 100 may acquire the aforementioned Intra Oral Scan (IOS) image 15, 3D face image 25, and CT scan image 35 from the 3-dimensional intraoral scanner 10, the 3-dimensional face capturing device 20, and the CT device 30, respectively. However, at least one of the Intra Oral Scan (IOS) image 15, the 3D face image 25, and the CT scan image 35 may also be acquired by the acquisition module 130 in the form of downloading what is stored in another storage medium or on the cloud.

The Intra Oral Scan (IOS) image 15, the 3D face image 25, and the CT scan image 35 acquired by the acquisition module 130 may be stored in the memory 110 of the dental registration data generation device 100. In the memory 110, the Intra Oral Scan (IOS) image 15, the 3D face image 25, and the CT scan image 35 may be stored separately for each patient, and if there are multiple data of the same type captured at different times, they may also be stored separately for each time period. In addition, the memory 110 may store personal information of each patient, treatment data such as a dentist's comments, and various applications including an application for generating dental registration data.

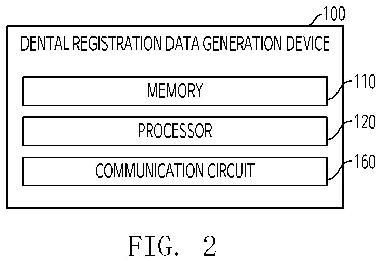

FIG. 2 is a block diagram of a dental registration data generation device 100 according to an embodiment of the present disclosure.

Referring to FIG. 2, according to an embodiment, the dental registration data generation device 100 may be a device that registers an intraoral scan image and a CT scan image. The dental registration data generation device 100 according to an embodiment may include a memory 110, a processor 120, and a communication circuit 160. The processor 120 of the dental registration data generation device 100 may execute software (e.g., a program) to control at least one other component (e.g., a hardware component, a software component) of the dental registration data generation device 100 connected to the processor 120, and may perform various data processing or operations. As at least part of the data processing or operations, the processor 120 may load instructions or data received from other components into the memory 110, process the instructions or data stored in the memory 110, and store the resulting data in the non-volatile memory 110. The memory 110 of the dental registration data generation device 100 may store information related to the above-described method or store a program in which the above-described method is implemented. The memory 110 may be a volatile memory or a non-volatile memory.

The processor 120 of the dental registration data generation device 100 according to an embodiment may execute a program and control the dental registration data generation device 100. The code of the program executed by the processor 120 may be stored in the memory 110. The dental registration data generation device 100 may be connected to an external device (e.g., a 3-dimensional intraoral scanner 10, a 3-dimensional face capturing device 20, a CT scanning device 30) through an input/output device (not shown in the drawing), and may exchange data. The processor 120 may be operatively connected to the components of the dental registration data generation device 100. The processor 120 may load instructions or data received from other components of the dental registration data generation device 100 into the memory 110, process the instructions or data stored in the memory 110, and store the resulting data.

The communication circuit 160 of the dental registration data generation device 100 according to an embodiment may establish a communication channel with an external device (e.g., a 3-dimensional intraoral scanner 10, a 3-dimensional face scanner 20, a CT scanning device 30) and transmit and receive various data with the external device. According to various embodiments, the communication circuit 160 may include a cellular communication module and be configured to connect to a cellular network (e.g., 3G, LTE, 5G, Wibro, or Wimax). According to various embodiments, the communication circuit 160 may include a short-range communication module and transmit and receive data with an external device using short-range communication (for example, Wi-Fi, Bluetooth, Bluetooth Low Energy (BLE), UWB), but is not limited thereto.

According to an embodiment, the dental registration data generation device 100 may further include an input device (not shown). The input device may receive commands or data to be used for the components of the server device from the outside. The input device may include, for example, a microphone, a mouse, or a keyboard.

According to an embodiment, the dental registration data generation device 100 may further include a display (not shown). The display may display various screens under the control of the processor 120.

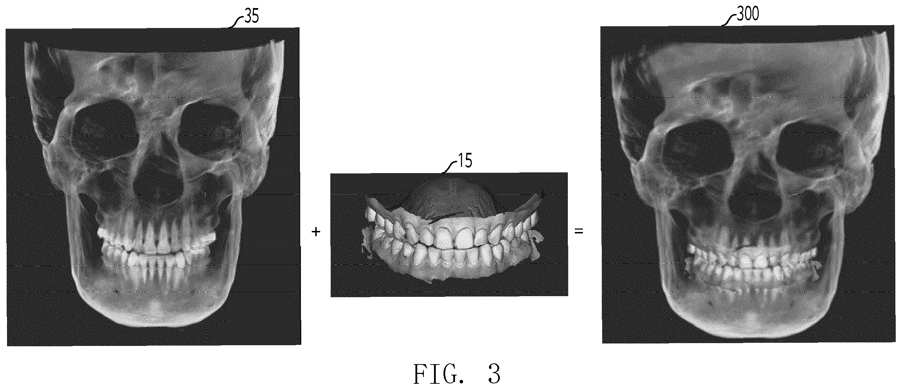

FIG. 3 is a diagram for schematically explaining the principle of generating dental registration data according to an embodiment of the present disclosure.

Referring to FIGS. 1 and 2, the registration module 140 may generate registration data by registering the Intra Oral Scan (IOS) image 15 and the CT scan image 35. The intraoral scan image may include a maxillary (upper teeth) scan image and a mandibular (lower teeth) scan image. The registration module 140 may generate two types of registration data in two ways according to the purpose of treatment. A specific method for generating the two types of registration data will be described later.

In addition, the registration module 140 may generate a dental 3D avatar as final registration data by registering the 3D face image 25 with the CT scan image 35 or the aforementioned registration data. According to an embodiment, for this registration, a method of extracting landmarks of a patient's 3-dimensional data (e.g., data of the face and/or dental region) and performing registration between images using the landmarks may be used. However, a method other than the method using landmarks may also be used, and it is not limited thereto.

According to an embodiment, the registration module 140 may extract three or more landmarks from the patient's 3-dimensional data. The Intra Oral Scan (IOS) image 15, the 3D face image 25, and the CT scan image 35 according to the embodiments of the present disclosure are all 3-dimensional or at least three or more are 3-dimensional data. By using three or more landmarks, all x, y, and z axes can be registered in the 3-dimensional data.

According to the method for generating dental registration data according to embodiments of the present disclosure, the extraction of landmarks may be manually designated by a user such as a dentist or a dental laboratory manager. For example, a user may designate landmarks using a pointer such as a mouse through a user interface of an application for generating dental registration data presented to the user by the display module 150. According to another embodiment, three or more landmarks may be extracted by the application for generating dental registration data.

Meanwhile, according to another embodiment, the application for generating dental registration data may be embedded with an artificial neural network module pre-trained with a large amount of data or connected to such an artificial neural network module via a network, thereby also enabling the extraction of landmarks based on artificial intelligence.

According to an embodiment, in the case of the Intra Oral Scan (IOS) image 15, since it corresponds to a 3D image generated by scanning the inside of the patient's oral cavity and does not include face data, landmarks of the dental region may be utilized for registration with other images 25, 35. In addition, when registering the 3D face image 25 with the CT scan image 35, landmarks of the face area excluding the patient's dental region may be used, or both landmarks of the patient's dental region and landmarks of the face area may be used.

The display module 150 may display the registration data generated by the registration module 140 through a display. The display module 150 may display a dental 3D avatar generated by the registration module 140 through the display of the device 100. According to an embodiment, the display module 150 may display the registration data on a user interface provided by an application for generating dental registration data. In addition, the display module 150 may allow a user such as a dentist or a dental laboratory manager to see an appropriate representation as needed by adjusting the transparency of some of the three images 15, 25, 35 constituting the dental 3D avatar or making it possible to select whether to display them. In addition, the display module 150 may allow the user to adjust the position or shape of some data, for example, the intraoral scan image 15, in the dental 3D avatar displayed. In addition, the display module 150 may display reference lines or reference planes such as a Mid-line, Alar line, Occlusal Plane, Campers Plane, etc., along with the dental 3D avatar so that a user can utilize them for treatment and consultation for the patient.

### [Method for Generating Dental Registration Data]

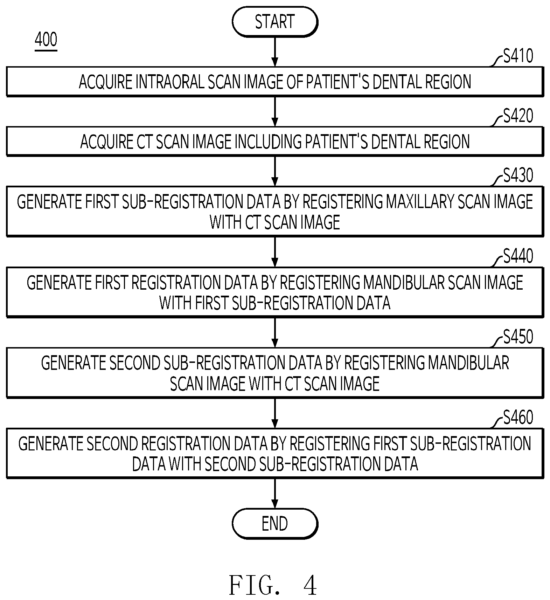

FIG. 4 is a flowchart for explaining a method for generating dental registration data according to an embodiment of the present disclosure.

First, referring to FIG. 4, in the method for generating dental registration data according to an embodiment of the present disclosure, the processor 120 of the dental registration data generation device 100 may, in step S410, acquire an Intra Oral Scan (IOS) image of a patient's dental region. The intraoral scan image may include a maxillary scan image and a mandibular scan image. For example, a user (e.g., a dentist, a dental technician) may acquire an intraoral scan image of a patient's dental region using a 3-dimensional intraoral scanner 20. The user may acquire a maxillary scan image of the patient's maxillary dental region using the 3-dimensional intraoral scanner 20. The user may acquire a mandibular scan image of the patient's mandibular dental region using the 3-dimensional intraoral scanner 20.

The dental registration data generation device 100 may acquire a maxillary scan image and a mandibular scan image acquired from the 3-dimensional intraoral scanner 20. Alternatively, the dental registration data generation device 100 may also acquire the maxillary scan image and the mandibular scan image by downloading them from another storage medium or from the cloud. The dental registration data generation device 100 may generate an intraoral scan image by registering the maxillary scan image and the mandibular scan image with each other. The intraoral scan image in which the maxillary scan image and the mandibular scan image are registered with each other in this way is a state in which the patient has occluded the oral cavity in a normal state, and the occlusion state of such an intraoral scan image (the state in which the patient has bitten down) may be called an intraoral scan occlusion state (or oral occlusion state).

The processor 120 according to an embodiment may, in step S420, acquire a CT scan image including the patient's dental region. For example, a user may acquire a CT scan image including the patient's dental region using a CT scanning device 30. The patient may perform CT scanning while being fixed with a mouthpiece (or bite block) of the CT scanning device 30 in his or her mouth. The occlusion state of the oral cavity during CT scanning in this way may be called a CT occlusion state.

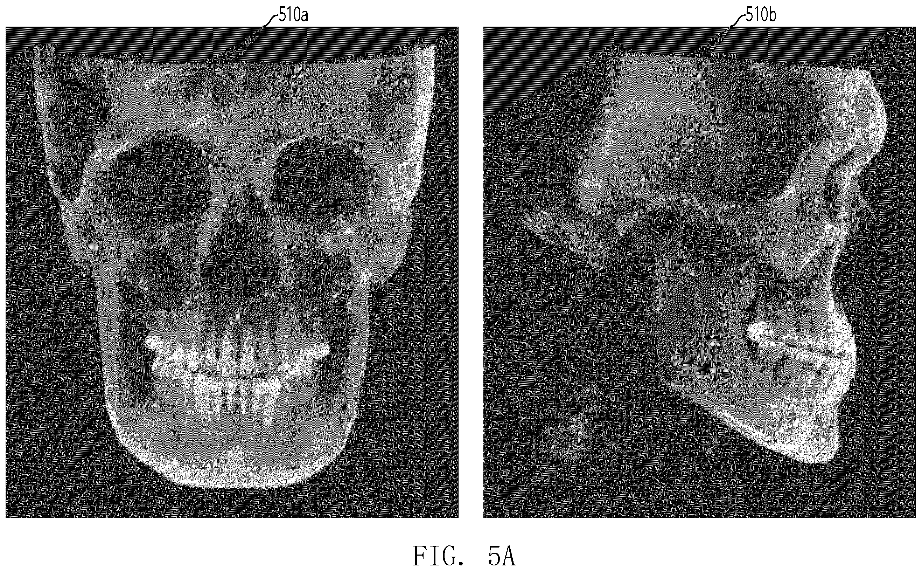

The processor 120 according to an embodiment may, in step S430, generate first sub-registration data by registering the maxillary scan image with the CT scan image. The first sub-registration data is necessary to generate both first registration data corresponding to the intraoral scan occlusion state and second registration data corresponding to the CT occlusion state. A method for generating the first sub-registration data will be described with reference to FIGS. 5A to 5C.

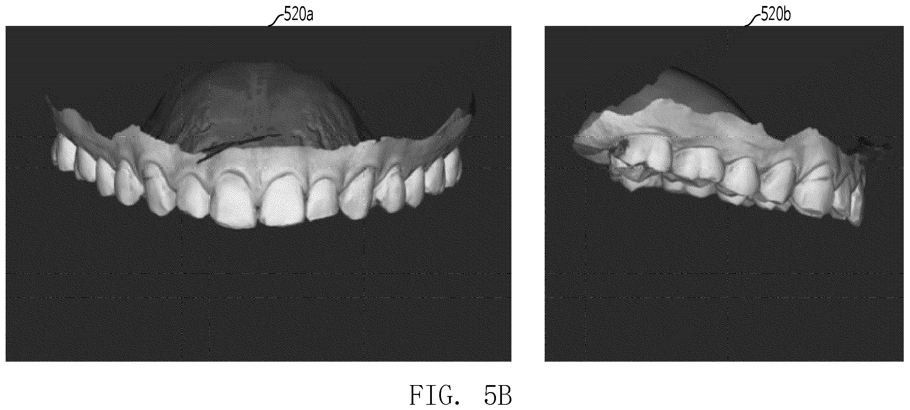

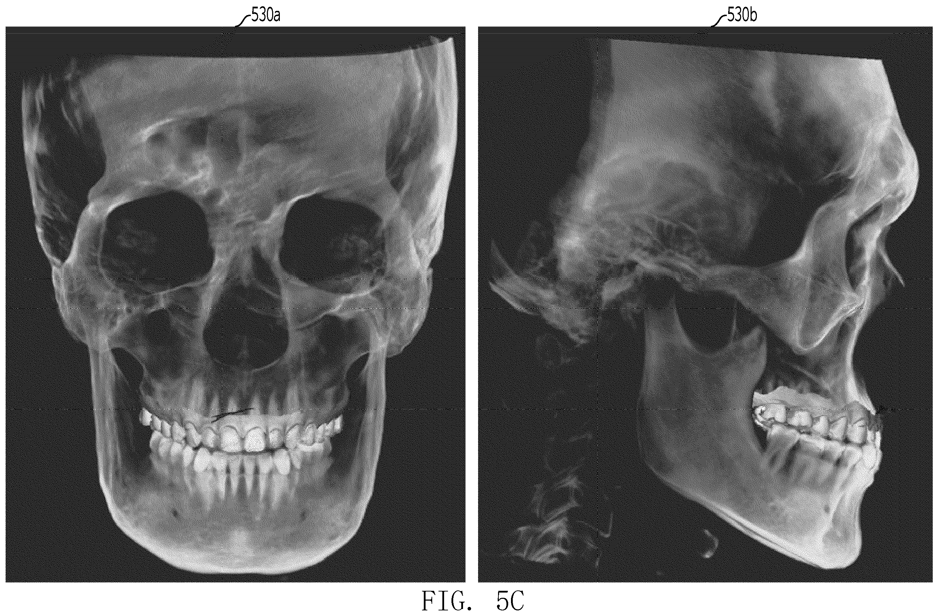

FIG. 5A is a CT scan image including a patient's dental region. The CT scan image may be a 3-dimensional CT scan image. The image 510a of FIG. 5A may be a CT scan image viewed from the front, and the image 510b may be a CT scan image viewed from the side. FIG. 5B is a maxillary scan image of a patient's maxillary dental region. The maxillary scan image may be a 3-dimensional intraoral scan image. The image 520a of FIG. 5B may be a maxillary scan image viewed from the front, and the image 520b of FIG. 5B may be a maxillary scan image viewed from the side. FIG. 5C is first sub-registration data in which the maxillary scan image is registered with the CT scan image. The first sub-registration data of FIG. 5C may be 3-dimensional data generated by registering a 3-dimensional maxillary scan image and a 3-dimensional CT scan image. The image 530a of FIG. 5C may be first sub-registration data viewed from the front, and the image 530b may be first sub-registration data viewed from the side.

Referring to FIGS. 5A to 5C, the processor 120 according to an embodiment may generate the first sub-registration data by registering the maxillary scan image and the CT scan image based on at least three landmarks. Since each image corresponds to 3-dimensional data, the number of landmarks used for generating the first sub-registration data may be three or more, and they may be extracted from the patient's face and/or dental region. For example, the processor 120 may extract three landmarks from the CT scan image and extract three corresponding landmarks from the maxillary scan data. The landmarks may, for example, indicate points located on the maxillary teeth and gingiva. However, the positions of the landmarks described above are exemplary, and they may also be extracted from various positions such as the middle part of the front teeth and points located on the boundary line between teeth. For generating registration data for use in dental treatment, precise registration of the dental region is important, so it is necessary to accurately extract landmarks of the patient's dental region. Therefore, if possible, if precise registration is possible with only landmarks of the patient's dental region, it is more effective because there is no need to additionally extract landmarks from the facial area other than the patient's teeth.

The processor 120 according to an embodiment may generate the first sub-registration data from the maxillary scan image and the CT scan image using a first deep learning model. The first deep learning model may be an artificial intelligence model constructed by modeling a correlation between input data and output data according to a deep learning algorithm, where the input data is a plurality of maxillary scan images and a plurality of CT scan image sets for a plurality of patients, and the output data is a plurality of sub-registration data sets in which each of the plurality of maxillary scan images is registered with each of the plurality of CT scan images.

In the present disclosure, a deep learning algorithm may mean that computer software improves its data processing ability through learning using data and experience of processing data. Deep learning may be performed by a deep learning model. The deep learning model is constructed by modeling the correlation between data, and the correlation may be expressed by a plurality of parameters. A deep learning model extracts and analyzes features from given data to derive a correlation between data, and deep learning can be said to be the process of optimizing the parameters of the deep learning model by repeating this process. For example, when data is given as an input and output pair of a deep learning model, the deep learning model can learn the mapping (correlation) between the input and output. Alternatively, even when only input data is given, the learning model can derive the regularity between the given data and learn the relationship. In an embodiment, the deep learning algorithm may be at least one selected from a deep neural network, a recurrent neural network, a convolution neural network, a machine learning model for classification-regression analysis, a reinforcement learning model, decision tree learning, association rule learning, genetic programming, inductive logic programming, a support vector machine, clustering, a Bayesian network, or identity metric learning.

Through the process described above, by registering the maxillary scan image disclosed in FIG. 5B with the CT scan image disclosed in FIG. 5A, the first sub-registration data disclosed in FIG. 5C may be generated.

The processor 120 according to an embodiment may, in step S440, generate first registration data by registering the mandibular scan image with the first sub-registration data. The first registration data may mean registration data according to the intraoral scan occlusion state. Specifically, the processor 120 may generate the first registration data by registering the mandibular scan image with the maxillary scan image on the first sub-registration data. For example, the processor 120 may register the mandibular scan image and the maxillary scan image on the first sub-registration data so that they are in an intraoral scan occlusion state. The processor 120 may register the mandibular scan image with the first sub-registration data by moving the mandibular scan image along with the maxillary scan image by the amount the maxillary scan image is aligned with the CT scan image.

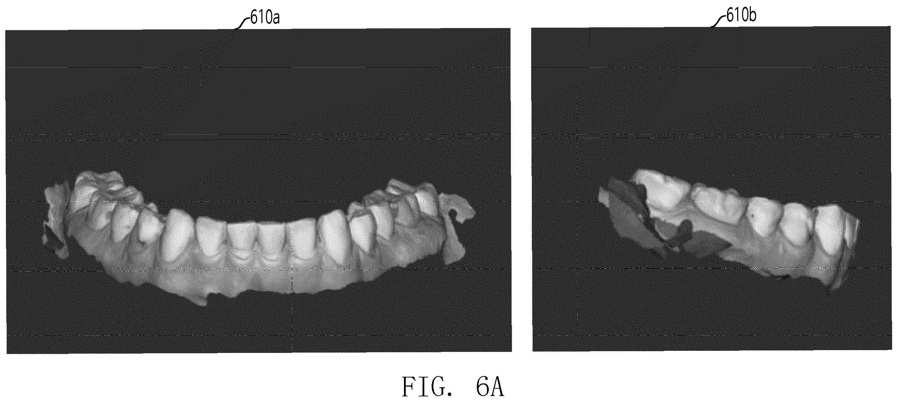

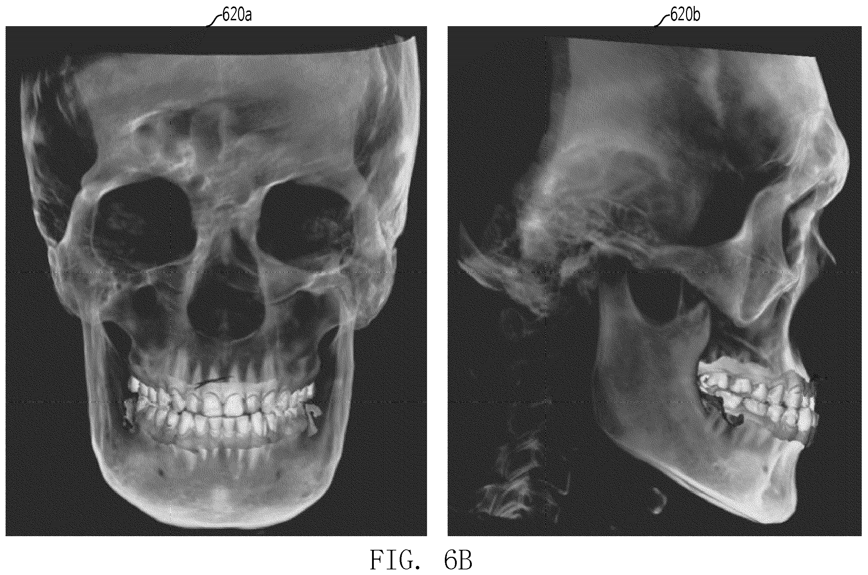

FIGS. 6A and 6B are diagrams for explaining a method of generating first registration data. Specifically, FIG. 6A is a mandibular scan image obtained by scanning a patient's mandibular dental region, and FIG. 6B is a diagram showing first registration data. The image 610a of FIG. 6A is a mandibular scan image viewed from the front, and the image 610b is a mandibular scan image viewed from the side. The image 620a of FIG. 6B is first registration data viewed from the front, and the image 620b is first registration data viewed from the side.

Referring to FIGS. 6A and 6B, the processor 120 may generate the first registration data by registering the mandibular scan image of FIG. 6A with the first sub-registration data of FIG. 5C. The processor 120 may generate the first registration data by registering the mandibular scan image and the maxillary scan image on the first sub-registration data so that they are in an intraoral scan occlusion state. Since the first registration data is registration data generated by aligning the mandibular scan image based on the maxillary scan image of the first sub-registration data, the first registration data may represent the intraoral scan occlusion state. Therefore, for dental treatment that requires checking the intraoral scan occlusion state (for example, prosthetic treatment such as crowns), the first registration data may be used.

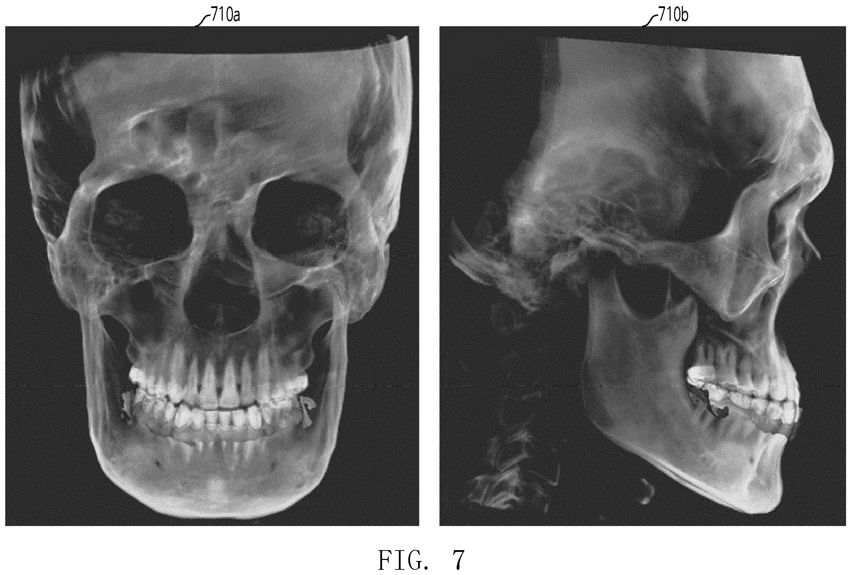

The processor 120 according to an embodiment may, in step S450, generate second sub-registration data by registering the mandibular scan image with the CT scan image. The second sub-registration data is necessary to generate second registration data corresponding to the CT occlusion state. A method of generating the second sub-registration data will be described with reference to FIGS. 5A, 6A, and 7. As described above, FIG. 5A is a CT scan image including the patient's dental region, and FIG. 6A is a mandibular scan image obtained by scanning the patient's mandibular dental region. FIG. 7 is a diagram showing second sub-registration data. The image 710a of FIG. 7 is second sub-registration data viewed from the front, and the image 710b is second sub-registration data viewed from the side.

Referring to FIGS. 5A, 6A, and 7, the processor 120 may generate second sub-registration data by registering the mandibular scan image of FIG. 6A with the CT scan image of FIG. 5A. According to an embodiment, the processor 120 may generate the second sub-registration data by registering the mandibular scan image and the CT scan image based on at least three landmarks. For example, the processor 120 may extract three landmarks from the CT scan image and extract three corresponding landmarks from the mandibular scan data. The landmarks may, for example, indicate predetermined points related to the mandibular teeth and gingiva. However, the positions of the landmarks described above are exemplary, and they may also be extracted from various positions such as the middle part of the front teeth and points located on the boundary line between teeth.

The processor 120 according to an embodiment may generate the second sub-registration data from the mandibular scan image and the CT scan image using a second deep learning model. The second deep learning model may be an artificial intelligence model constructed by modeling a correlation between input data and output data according to a deep learning algorithm, where the input data is a plurality of mandibular scan images and a plurality of CT scan image sets for a plurality of patients, and the output data is a plurality of sub-registration data sets in which each of the plurality of mandibular scan images is registered with each of the plurality of CT scan images.

In the present disclosure, a deep learning algorithm may mean that computer software improves its data processing ability through learning using data and experience of processing data. Deep learning may be performed by a deep learning model. The deep learning model is constructed by modeling the correlation between data, and the correlation may be expressed by a plurality of parameters. A deep learning model extracts and analyzes features from given data to derive a correlation between data, and deep learning can be said to be the process of optimizing the parameters of the deep learning model by repeating this process. For example, when data is given as an input and output pair of a deep learning model, the deep learning model can learn the mapping (correlation) between the input and output. Alternatively, even when only input data is given, the learning model can derive the regularity between the given data and learn the relationship. In an embodiment, the deep learning algorithm may be at least one selected from a deep neural network, a recurrent neural network, a convolution neural network, a machine learning model for classification-regression analysis, a reinforcement learning model, decision tree learning, association rule learning, genetic programming, inductive logic programming, a support vector machine, clustering, a Bayesian network, or identity metric learning.

Through the process described above, by registering the mandibular scan image disclosed in FIG. 6A with the CT scan image disclosed in FIG. 5A, the second sub-registration data disclosed in FIG. 7 may be generated.

The processor 120 according to an embodiment may, in step S460, generate second registration data by registering the first sub-registration data with the second sub-registration data. The second registration data may mean registration data according to the CT occlusion state. Specifically, since the CT scan image of the first sub-registration data and the CT scan image of the second sub-registration data are the same, the processor 120 may perform registration based on the CT scan image of the first sub-registration data and the CT scan image of the second sub-registration data. That is, the processor 120 may register the first sub-registration data and the second sub-registration data with each other so as to be in a CT occlusion state.

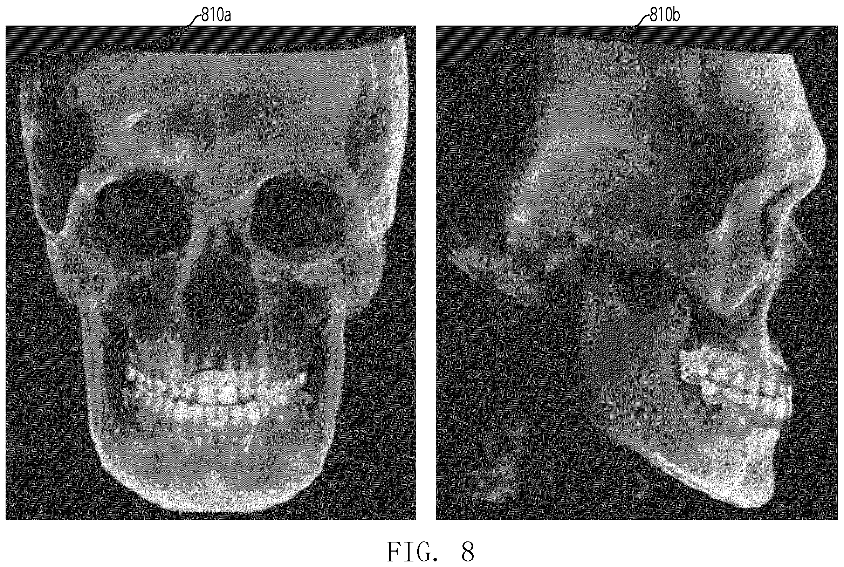

FIG. 8 is a diagram showing second registration data. The image 810a of FIG. 8 is second registration data viewed from the front, and the image 810b is second registration data viewed from the side. The process of generating the second registration data will be described with reference to FIGS. 5C, 7, and 8. The processor 120 may generate the second registration data of FIG. 8 by registering the first sub-registration data of FIG. 5C and the second sub-registration data of FIG. 7 with each other. The processor 120 may generate the second registration data by registering the CT scan image of the first sub-registration data and the CT scan image of the second sub-registration data so that they overlap.

A user may select and use the necessary registration data from the first registration data and the second registration data according to the purpose of treatment. In an embodiment, the processor 120 of the dental registration data generation device 100 may receive a user input for selecting one of the first registration data and the second registration data through the input device 100. The processor 120 may display the selected one registration data through a display in response to receiving the user input. In another embodiment, the processor 120 of the dental registration data generation device 100 may receive a user input regarding a treatment method for the patient's dental region through the input device 100. The processor 120 may select one of the first registration data and the second registration data corresponding to the user input. In addition, the processor 120 may display the selected one registration data through the display.

The processor 120 according to an embodiment may store the first registration data and the second registration data in the memory 110. Alternatively, the processor 120 may store the first registration data and the second registration data in the cloud.

The processor 120 according to an embodiment may acquire a 3-dimensional face image for a face including a patient's dental region. The processor 120 may generate a dental 3D avatar as final registration data by registering the 3-dimensional face image with one of the CT scan image, the first registration data, and the second registration data. For example, the processor 120 may register the 3-dimensional face image with the CT scan image, register the 3-dimensional face image with the first registration data, or register the 3-dimensional face image with the second registration data. In order to generate the dental 3D avatar, the processor 120 may extract landmarks corresponding to the landmarks extracted to generate the first registration data from the 3-dimensional face image. For example, landmarks corresponding to at least three landmarks extracted to generate the first sub-registration data may be extracted from the 3-dimensional face image.

As described above, according to a method for generating dental registration data according to another embodiment of the present disclosure, first sub-registration data generated based on registering a maxillary scan image among intraoral scan images with a CT scan image acquired in an intraoral occlusion state of a patient, and first registration data generated based on registering a mandibular scan image may be generated. In addition, according to a method for generating dental registration data according to another embodiment of the present disclosure, second registration data generated based on registering first sub-registration data generated based on registering a maxillary scan image among intraoral scan images with a CT scan image acquired in an intraoral occlusion state of a patient, and second sub-registration data generated based on registering a mandibular scan image with the CT scan image may be generated.

That is, since two types of registration data according to two occlusion states are all generated, not only can a user selectively utilize necessary data according to the purpose of a patient's dental treatment, but also an effect occurs in that the state of the teeth when the patient maintains an intraoral occlusion state can be more effectively expressed.

More specifically, depending on the treatment method, the first registration data may be more appropriate, the second registration data may be more appropriate, or both may be needed. For example, when designing a prosthesis such as a Crown/Inlay/Onlay, the occlusal relationship with other teeth is more important, so the first registration data according to the intraoral scan occlusion state can be utilized. On the other hand, when designing a Surgical Guide, it is necessary to check the patient's tooth root or nerve position, so the second registration data, which is data of the patient's dental occlusion state based on the CT scan image (i.e., the CT occlusion state), may be more appropriate. In addition, when designing a prosthesis and a surgical guide at the same time, both types of data (i.e., a first dental 3D avatar and a second dental 3D avatar) may be needed.

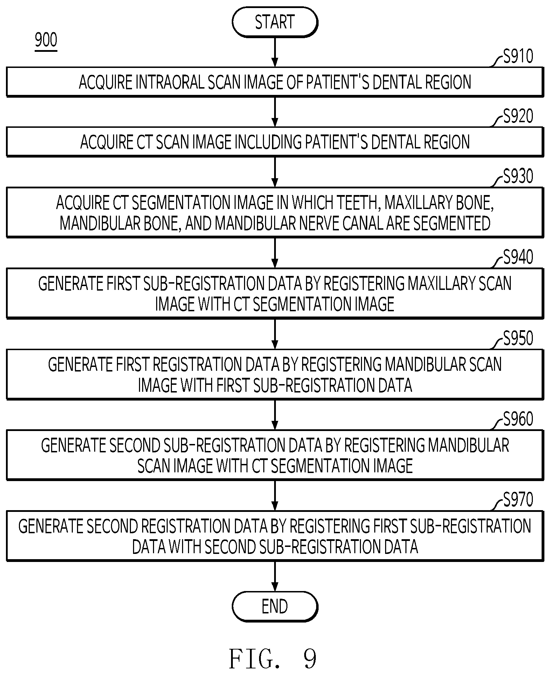

FIG. 9 is an operational flowchart of a dental registration data generation device 100 according to an embodiment of the present disclosure. Content that overlaps with the content described in FIG. 4 will be omitted.

Referring to the operational flowchart 900, the processor 120 of the dental registration data generation device 100 according to an embodiment may, in step S910, acquire an intraoral scan image of a patient's dental region. The processor 120 according to an embodiment may, in step S920, acquire a CT scan image including the patient's dental region.

The processor 120 according to an embodiment may, in step S930, acquire a CT segmentation image in which at least one of maxillary teeth, mandibular teeth, a maxillary bone, a mandibular bone, and a mandibular nerve canal is segmented by performing image processing on the CT scan image. The processor 120 may identify and extract at least one of the maxillary teeth, mandibular teeth, maxillary bone, mandibular bone, and mandibular nerve canal from the CT scan image. When creating data in a mesh format, each part can be segmented in the CT occlusion state as it is.

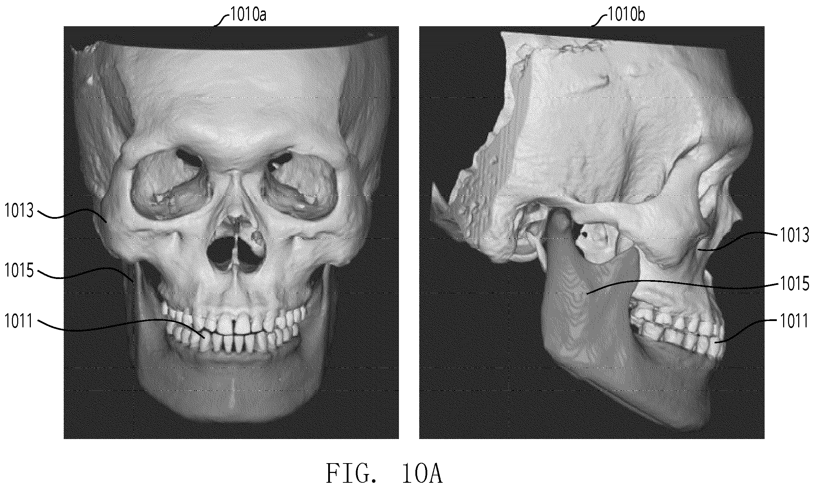

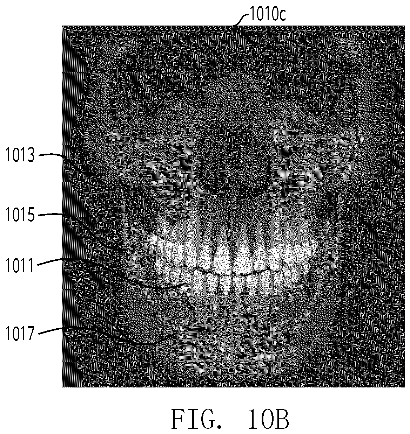

FIGS. 10A and 10B are diagrams showing a CT segmentation image. The image 1010a of FIG. 10A is a CT segmentation image viewed from the front, and the image 1010b is a CT segmentation image viewed from the side. The image 1010c of FIG. 10B is a semi-transparently displayed image of the image 1010a of FIG. 10A.

Referring to FIGS. 10A to 10c, the CT segmentation image may include teeth 1011, a maxillary bone 1013, a mandibular bone 1015, and a mandibular nerve canal 1017. That is, the processor 120 may identify and extract the teeth 1011, the maxillary bone 1013, the mandibular bone 1015, and the mandibular nerve canal 1017 from the CT scan image.

Returning to FIG. 9, the processor 120 according to an embodiment may, in step S940, generate first sub-registration data by registering the maxillary scan image with the CT segmentation image. The processor 120 according to an embodiment may, in step S950, generate first registration data by registering the mandibular scan image with the first sub-registration data. The difference from the method disclosed in FIG. 4 is that the method of FIG. 9 uses a CT segmentation image in which the teeth, maxillary bone, mandibular bone, and/or mandibular nerve canal are segmented for registration, instead of using the CT scan image as it is.

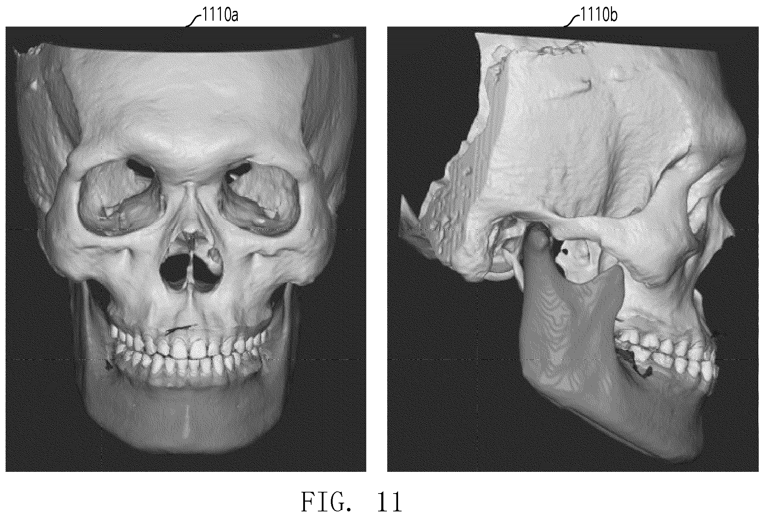

FIG. 11 is a diagram showing first registration data according to an embodiment. The image 1110a of FIG. 11 is first registration data viewed from the front, and the image 1110b is first registration data viewed from the side. That is, the processor 120 may register the maxillary scan image and the mandibular scan image with the CT segmentation image in order. At this time, the processor 120 may register the mandibular scan image based on the maxillary scan image of the first sub-registration data. That is, the processor 120 may generate the first registration data by moving the mandibular scan image by the amount the maxillary scan data is aligned with the CT segmentation image. Therefore, the first registration data may represent the intraoral scan occlusion state.

According to an embodiment, the segmented mandibular bone, mandibular teeth, and mandibular nerve canal may be adjusted to the intraoral scan occlusion state. The processor 120 may calculate a transformation relationship between the first registration data and the second sub-registration data. For example, the processor 120 may generate second sub-registration data by registering the mandibular scan image of the first registration data with the CT segmentation image. The processor 120 may calculate a transformation matrix from the first registration data to the second sub-registration data. The processor 120 may calculate an inverse matrix for the transformation matrix. The processor 120 may accurately calculate and express the positions of the segmented mandibular bone, mandibular teeth, and mandibular nerve canal in the intraoral scan occlusion state by applying the inverse transformation matrix to the segmented mandibular bone, mandibular teeth, and mandibular nerve canal.

Returning to FIG. 9, the processor 120 according to an embodiment may, in step S960, generate second sub-registration data by registering the mandibular scan image with the CT segmentation image. The processor 120 according to an embodiment may, in step S970, generate second registration data by registering the first sub-registration data with the second sub-registration data. The difference from the method disclosed in FIG. 4 is that the method of FIG. 9 uses a CT segmentation image in which the teeth, maxillary bone, mandibular bone, and/or mandibular nerve canal are segmented for registration, instead of using the CT scan image as it is.

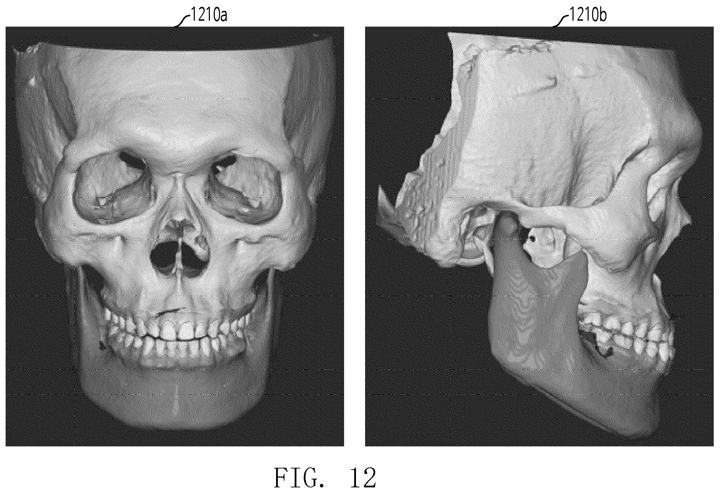

FIG. 12 is a diagram showing second registration data according to an embodiment. The image 1210a of FIG. 12 is second registration data viewed from the front, and the image 1210b is first registration data viewed from the side. That is, the processor 120 may generate first sub-registration data by registering the maxillary scan image with the CT segmentation image, and generate second sub-registration data by registering the mandibular scan image with the CT segmentation image. At this time, the processor 120 may register the first sub-registration data and the second sub-registration data based on the CT segmentation image. Therefore, the second registration data may represent the CT occlusion state.

### [Computer-Readable Recording Medium]

It is self-evident that each step or operation of the method according to the embodiments of the present disclosure may be performed by a computer including one or more processors upon execution of a computer program stored in a computer-readable recording medium.

The computer-executable instructions stored in the aforementioned recording medium can be implemented through a computer program programmed to perform each corresponding step, and this computer program can be stored in a computer-readable recording medium and executed by a processor. The computer-readable recording medium may be a non-transitory readable medium. Here, the non-transitory readable medium means a medium that stores data semi-permanently and is readable by a device, not a medium that stores data for a short period of time, such as a register, cache, or memory. Specifically, programs for performing the various methods described above may be stored and provided in non-transitory readable media such as semiconductor memory devices like EPROM (Erasable Programmable Read-Only Memory), EEPROM (Electrically Erasable Programmable Read-Only Memory), and flash memory devices, magnetic disks like internal hard disks and removable disks, optical-magnetic disks, and non-volatile memories including CD-ROM and DVD-ROM disks.

The method according to the various examples disclosed in this document may be provided included in a computer program product. The computer program product may be distributed in the form of a machine-readable storage medium (e.g., compact disc read only memory (CD-ROM)), or online through an application store (e.g., Play StoreTM). In the case of online distribution, at least part of the computer program product may be at least temporarily stored in a storage medium such as the memory of a manufacturer's server, an application store's server, or a relay server, or may be temporarily generated.

As described above, a person of ordinary skill in the art to which the present disclosure pertains will be able to understand that the present disclosure can be implemented in other specific forms without changing its essential features. Therefore, the above-described embodiments should be understood as illustrative in all respects and not restrictive. The scope of the present disclosure is indicated by the appended claims rather than the detailed description, and all changes or modified forms derived from the meaning and scope of the claims and their equivalent concepts should be interpreted as being included in the scope of the present disclosure.

It should be noted that the features and advantages described in this specification are not exhaustive, and in particular, many additional features and advantages will be apparent to those skilled in the art in consideration of the drawings, specification, and claims. Furthermore, it should be noted that the language used in this specification was chosen primarily for ease of reading and for teaching purposes, and may not have been chosen to delineate or limit the subject matter of the present disclosure.

The foregoing description of the embodiments of the present disclosure has been presented for purposes of illustration. It is not intended to be exhaustive or to limit the present disclosure to the precise form disclosed. Many modifications and variations are possible in light of the above teaching, as will be understood by those skilled in the art.

Therefore, the scope of the present disclosure is not limited by the detailed description, but by any claims of an application based thereon. Accordingly, the disclosure of the embodiments of the present disclosure is illustrative and not intended to limit the scope of the present disclosure described in the following claims.

### Explanation of reference symbols

10: 3-dimensional intraoral scanner
15: Intraoral scan (IOS) image
20: 3-dimensional face capturing device
25: 3D face image
30: Dental CT device
35: CT scan image
100: Dental registration data generation device
110: Memory
120: Processor
130: Acquisition module
140: Registration module
150: Display module

## Claims

1. A method for generating dental registration data by a device comprising a communication circuit, a memory, and a processor,
the method performed by the processor comprising:
acquiring an intraoral scan image of a dental region of a patient, wherein the intraoral scan image comprises a maxillary scan image and a mandibular scan image;
acquiring a CT scan image including the dental region of the patient;
generating first sub-registration data by registering the maxillary scan image with the CT scan image;
generating first registration data by registering the mandibular scan image with the first sub-registration data;
generating second sub-registration data by registering the mandibular scan image with the CT scan image; and
generating second registration data by registering the first sub-registration data with the second sub-registration data.

2. The method as claimed in claim 1,
further comprising obtaining a CT segmentation image in which at least one of maxillary teeth, mandibular teeth, maxillary bone, mandibular bone, and a mandibular nerve canal is segmented by the processor performing image processing on the CT scan image.

3. The method as claimed in claim 2,
wherein generating the first sub-registration data comprises:
generating the first sub-registration data by registering the maxillary scan image with the CT segmentation image, and
generating the second sub-registration data comprises:
generating the second sub-registration data by registering the mandibular scan image with the CT segmentation image.

4. The method as claimed in claim 3,
wherein generating the first registration data comprises:
calculating a transformation matrix from the first registration data to the second sub-registration data; and
applying an inverse transformation matrix corresponding to the transformation matrix to at least one of the segmented mandibular teeth, the segmented mandibular bone, and the segmented mandibular nerve canal.

5. The method as claimed in claim 1,
wherein generating the first sub-registration data comprises:
generating the first sub-registration data from the maxillary scan image and the CT scan image using a first deep learning model, and
the first deep learning model is an artificial intelligence model constructed by modeling a correlation between input data and output data according to a deep learning algorithm, the input data comprising a plurality of maxillary scan images and a plurality of sets of CT scan images from a plurality of patients, and the output data comprising a plurality of sets of sub-registration data generated by registering each of the plurality of maxillary scan images with each of the plurality of CT scan images.

6. The method as claimed in claim 1,
wherein generating the second sub-registration data comprises:
generating the second sub-registration data from the mandibular scan image and the CT scan image using a second deep learning model, and
the second deep learning model is an artificial intelligence model constructed by modeling a correlation between input data and output data according to a deep learning algorithm, the input data comprising a plurality of mandibular scan images and a plurality of sets of CT scan images from a plurality of patients, and the output data comprising a plurality of sets of sub-registration data generated by registering each of the plurality of mandibular scan images with each of the plurality of CT scan images.

7. The method as claimed in claim 1,
wherein the first sub-registration data and the second sub-registration data are generated based on at least three landmarks of 3-dimensional data of the patient,
the at least three landmarks are extracted by an application for generating dental registration data,
the at least three landmarks are extracted using an artificial neural network module embedded in the application or connected through a network, and
the artificial neural network module is pretrained using pretraining data.

8. A device for generating dental registration data, comprising:
a communication circuit;
a memory; and
a processor,
wherein the processor is configured to:
acquire an intraoral scan image of a dental region of a patient, wherein the intraoral scan image comprises a maxillary scan image and a mandibular scan image,
acquire a CT scan image including the dental region of the patient,
generate first sub-registration data by registering the maxillary scan image with the CT scan image,
generate first registration data by registering the mandibular scan image with the first sub-registration data,
generate second sub-registration data by registering the mandibular scan image with the CT scan image, and
generate second registration data by registering the first sub-registration data with the second sub-registration data.

9. The device as claimed in claim 8,
further comprising:
an input device; and
a display,
wherein the processor is configured to:
receive a user input through the input device to select one registration data from the first registration data and the second registration data, and
display the selected one registration data on the display in response to receiving the user input.

10. The device as claimed in claim 8,
further comprising:
an input device; and
a display,
wherein the processor is configured to:
receive a user input through the input device regarding a treatment method for the dental region of the patient,
select one registration data corresponding to the user input from the first registration data and the second registration data, and
display the selected one registration data on the display.

11. The device as claimed in claim 8,
wherein the processor is configured to obtain a CT segmentation image in which at least one of maxillary teeth, mandibular teeth, maxillary bone, mandibular bone, and a mandibular nerve canal is segmented by performing image processing on the CT scan image.

12. The device as claimed in claim 11,
wherein the processor is configured to:
generate the first sub-registration data by registering the maxillary scan image with the CT segmentation image, and
generate the second sub-registration data by registering the mandibular scan image with the CT segmentation image.

13. The device as claimed in claim 12,
wherein the processor is configured to:
calculate a transformation matrix from the first registration data to the second sub-registration data, and
apply an inverse transformation matrix corresponding to the transformation matrix to at least one of the segmented mandibular teeth, the segmented mandibular bone, and the segmented mandibular nerve canal.

14. The device as claimed in claim 8,
wherein the processor is configured to generate the first sub-registration data from the maxillary scan image and the CT scan image using a first deep learning model,
the first deep learning model being an artificial intelligence model constructed by modeling a correlation between input data and output data according to a deep learning algorithm, the input data comprising a plurality of maxillary scan images and a plurality of sets of CT scan images from a plurality of patients, and the output data comprising a plurality of sets of sub-registration data generated by registering each of the plurality of maxillary scan images with each of the plurality of CT scan images.

15. The device as claimed in claim 8,
wherein the processor is configured to generate the second sub-registration data from the mandibular scan image and the CT scan image using a second deep learning model,
the second deep learning model being an artificial intelligence model constructed by modeling a correlation between input data and output data according to a deep learning algorithm, the input data comprising a plurality of mandibular scan images and a plurality of sets of CT scan images from a plurality of patients, and the output data comprising a plurality of sets of sub-registration data generated by registering each of the plurality of mandibular scan images with each of the plurality of CT scan images.

## Patentansprüche

1. Verfahren zur Erzeugung von Zahnregistrierungsdaten durch eine Vorrichtung, die eine Kommunikationsschaltung, einen Speicher und einen Prozessor umfasst,
wobei das von dem Prozessor durchgeführte Verfahren Folgendes umfasst:
Erfassen eines intraoralen Scanbildes eines Zahnbereichs eines Patienten, wobei das intraorale Scanbild ein Scanbild des Oberkiefers und ein Scanbild des Unterkiefers umfasst;
Erfassen eines CT-Scanbildes, das den Zahnbereich des Patienten umfasst;
Erzeugen von ersten Teilregistrierungsdaten durch Registrierung des Scanbildes des Oberkiefers mit dem CT-Scanbild;
Erzeugen von ersten Registrierungsdaten durch Registrierung des Scanbildes des Unterkiefers mit den ersten Teilregistrierungsdaten;
Erzeugen von zweiten Teilregistrierungsdaten durch Registrierung des Scanbildes des Unterkiefers mit dem CT-Scanbild; und
Erzeugen von zweiten Registrierungsdaten durch Registrierung der ersten Teilregistrierungsdaten mit den zweiten Teilregistrierungsdaten.

2. Verfahren nach Anspruch 1,
ferner umfassend das Erhalten eines CT-Segmentierungsbildes, in dem mindestens eines unter Oberkieferzähnen, Unterkieferzähnen, Oberkieferknochen, Unterkieferknochen und einem Unterkiefernervenkanal durch den Prozessor segmentiert wird, der die Bildverarbeitung an dem CT-Scanbild durchführt.

3. Verfahren nach Anspruch 2,
wobei das Erzeugen der ersten Teilregistrierungsdaten Folgendes umfasst:
Erzeugen der ersten Teilregistrierungsdaten durch Registrierung des Scanbildes des Oberkiefers mit dem CT-Segmentierungsbild sowie
das Erzeugen der zweiten Teilregistrierungsdaten umfasst:
Erzeugen der zweiten Teilregistrierungsdaten durch Registrierung des Scanbildes des Unterkiefers mit dem CT-Segmentierungsbild.

4. Verfahren nach Anspruch 3,
wobei das Erzeugen der ersten Registrierungsdaten Folgendes umfasst:
Berechnen einer Transformationsmatrix von den ersten Registrierungsdaten zu den zweiten Teilregistrierungsdaten; und
Anwenden einer der Transformationsmatrix entsprechenden inversen Transformationsmatrix auf mindestens einem der segmentierten Unterkieferzähne, des segmentierten Unterkieferknochens und des segmentierten Unterkiefernervenkanals.

5. Verfahren nach Anspruch 1,
wobei das Erzeugen der ersten Teilregistrierungsdaten Folgendes umfasst:
Erzeugen der ersten Teilregistrierungsdaten aus dem Scanbild des Oberkiefers und dem CT-Scanbild unter Verwendung eines ersten Deep-Learning-Modells sowie
das erste Deep-Learning-Modell ist ein Modell der künstlichen Intelligenz, das durch die Modellierung einer Korrelation zwischen Eingabedaten und Ausgabedaten gemäß einem Deep-Learning-Algorithmus erstellt wird, wobei die Eingabedaten eine Vielzahl von Scanbildern des Oberkiefers und eine Vielzahl von Sätzen von CT-Scanbildern von einer Vielzahl von Patienten umfassen und die Ausgabedaten eine Vielzahl von Sätzen von Teilregistrierungsdaten umfassen, die durch Registrierung jedes der Vielzahl von Scanbildern des Oberkiefers mit jedem der Vielzahl von CT-Scanbildern erzeugt werden.

6. Verfahren nach Anspruch 1,
wobei das Erzeugen der zweiten Teilregistrierungsdaten Folgendes umfasst:
Erzeugen der zweiten Teilregistrierungsdaten aus dem Scanbild des Unterkiefers und dem CT-Scanbild unter Verwendung eines zweiten Deep-Learning-Modells, und
das zweite Deep-Learning-Modell ist ein Modell der künstlichen Intelligenz, das durch die Modellierung einer Korrelation zwischen Eingabedaten und Ausgabedaten gemäß einem Deep-Learning-Algorithmus erstellt wird, wobei die Eingabedaten eine Vielzahl von Scanbildern des Unterkiefers und eine Vielzahl von Sätzen von CT-Scanbildern von einer Vielzahl von Patienten umfassen und die Ausgabedaten eine Vielzahl von Sätzen von Teilregistrierungsdaten umfassen, die durch Registrierung jedes der Vielzahl von Scanbildern des Unterkiefers mit jedem der Vielzahl von CT-Scanbildern erzeugt werden.

7. Verfahren nach Anspruch 1,
wobei die ersten Teilregistrierungsdaten und die zweiten Teilregistrierungsdaten auf Grundlage von mindestens drei Orientierungspunkten dreidimensionaler Daten des Patienten erzeugt werden,
die mindestens drei Orientierungspunkte durch eine Anwendung zur Erzeugung von Zahnregistrierungsdaten extrahiert werden,
die mindestens drei Orientierungspunkte unter Verwendung eines in die Anwendung integrierten oder über ein Netzwerk verbundenen Moduls eines künstlichen neuronalen Netzes extrahiert werden, und
das Modul des künstlichen neuronalen Netzes unter Verwendung von Vortrainingsdaten vortrainiert wird.

8. Vorrichtung zur Erzeugung von Zahnregistrierungsdaten, umfassend:
eine Kommunikationsschaltung;
einen Speicher; und
einen Prozessor,
wobei der Prozessor ausgelegt ist zum:
Erfassen eines intraoralen Scanbildes eines Zahnbereichs eines Patienten, wobei das intraorale Scanbild ein Scanbild des Oberkiefers und ein Scanbild des Unterkiefers umfasst;
Erfassen eines CT-Scanbildes, das den Zahnbereich des Patienten umfasst,
Erzeugen von ersten Teilregistrierungsdaten durch Registrierung des Scanbildes des Oberkiefers mit dem CT-Scanbild,
Erzeugen von ersten Registrierungsdaten durch Registrierung des Scanbildes des Unterkiefers mit den ersten Teilregistrierungsdaten,
Erzeugen von zweiten Teilregistrierungsdaten durch Registrierung des Scanbildes des Unterkiefers mit dem CT-Scanbild, und
Erzeugen von zweiten Registrierungsdaten durch Registrierung der ersten Teilregistrierungsdaten mit den zweiten Teilregistrierungsdaten.

9. Vorrichtung nach Anspruch 8,
ferner umfassend:
eine Eingabevorrichtung; und
eine Anzeige,
wobei der Prozessor ausgelegt ist zum:
Empfangen einer Benutzereingabe über die Eingabevorrichtung, um ein Registrierungsdatum aus den ersten Registrierungsdaten und den zweiten Registrierungsdaten auszuwählen, und
Anzeigen des ausgewählten einen Registrierungsdatums auf der Anzeige als Reaktion auf das Empfangen der Benutzereingabe.

10. Vorrichtung nach Anspruch 8, ferner umfassend:
eine Eingabevorrichtung; und
eine Anzeige,
wobei der Prozessor ausgelegt ist zum:
Empfangen einer Benutzereingabe über die Eingabevorrichtung in Bezug auf ein Behandlungsverfahren für den Zahnbereich des Patienten,
Auswählen von einem Registrierungsdatum, das der Benutzereingabe entspricht, aus den ersten Registrierungsdaten und den zweiten Registrierungsdaten, und
Anzeigen des ausgewählten Registrierungsdatums auf der Anzeige.

11. Vorrichtung nach Anspruch 8,
wobei der Prozessor dazu ausgelegt ist, ein CT-Segmentierungsbild zu erhalten, in dem mindestens eines unter Oberkieferzähnen, Unterkieferzähnen, Oberkieferknochen, Unterkieferknochen und einem Unterkiefernervenkanal segmentiert wird, indem die Bildverarbeitung an dem CT-Scanbild durchführt wird.

12. Vorrichtung nach Anspruch 11,
wobei der Prozessor ausgelegt ist zum:
Erzeugen der ersten Teilregistrierungsdaten durch Registrierung des Scanbildes des Oberkiefers mit dem CT-Segmentierungsbild sowie
Erzeugen der zweiten Teilregistrierungsdaten durch Registrierung des Scanbildes des Unterkiefers mit dem CT-Segmentierungsbild.

13. Vorrichtung nach Anspruch 12,
wobei der Prozessor ausgelegt ist zum:
Berechnen einer Transformationsmatrix von den ersten Registrierungsdaten zu den zweiten Teilregistrierungsdaten, und
Anwenden einer der Transformationsmatrix entsprechenden inversen Transformationsmatrix auf mindestens einem der segmentierten Unterkieferzähne, des segmentierten Unterkieferknochens und des segmentierten Unterkiefernervenkanals.

14. Vorrichtung nach Anspruch 8,
wobei der Prozessor dazu ausgelegt ist, die ersten Teilregistrierungsdaten aus dem Scanbild des Oberkiefers und dem CT-Scanbild unter Verwendung eines ersten Deep-Learning-Modells zu erzeugen,
wobei das erste Deep-Learning-Modell ein Modell der künstlichen Intelligenz ist, das durch die Modellierung einer Korrelation zwischen Eingabedaten und Ausgabedaten gemäß einem Deep-Learning-Algorithmus erstellt wird, wobei die Eingabedaten eine Vielzahl von Scanbildern des Oberkiefers und eine Vielzahl von Sätzen von CT-Scanbildern von einer Vielzahl von Patienten umfassen und die Ausgabedaten eine Vielzahl von Sätzen von Teilregistrierungsdaten umfassen, die durch Registrierung jedes der Vielzahl von Scanbildern des Oberkiefers mit jedem der Vielzahl von CT-Scanbildern erzeugt werden.

15. Vorrichtung nach Anspruch 8,
wobei der Prozessor dazu ausgelegt ist, die zweiten Teilregistrierungsdaten aus dem Scanbild des Unterkiefers und dem CT-Scanbild unter Verwendung eines zweiten Deep-Learning-Modells zu erzeugen,
wobei das zweite Deep-Learning-Modell ein Modell der künstlichen Intelligenz ist, das durch die Modellierung einer Korrelation zwischen Eingabedaten und Ausgabedaten gemäß einem Deep-Learning-Algorithmus erstellt wird, wobei die Eingabedaten eine Vielzahl von Scanbildern des Unterkiefers und eine Vielzahl von Sätzen von CT-Scanbildern von einer Vielzahl von Patienten umfassen und die Ausgabedaten eine Vielzahl von Sätzen von Teilregistrierungsdaten umfassen, die durch Registrierung jedes der Vielzahl von Scanbildern des Unterkiefers mit jedem der Vielzahl von CT-Scanbildern erzeugt werden.

## Revendications

1. Procédé de génération de données de recalage dentaire à l'aide d'un dispositif comprenant un circuit de communication, une mémoire et un processeur,
le procédé mis en œuvre par le processeur, comprenant :
l'acquisition d'une image de numérisation intra-orale d'une région dentaire d'un patient, l'image de numérisation intra-orale comprenant une image de numérisation de l'arcade maxillaire et une image de numérisation de l'arcade mandibulaire ;
l'acquisition d'une image de tomodensitométrie incluant la région dentaire du patient ;
la génération de premières données de sous-recalage en recalant l'image de numérisation de l'arcade maxillaire sur l'image de tomodensitométrie ;
la génération de premières données de recalage en recalant l'image de numérisation de l'arcade mandibulaire avec les premières données de sous-recalage ;
la génération de secondes données de sous-recalage en recalant l'image de numérisation de l'arcade mandibulaire sur l'image de tomodensitométrie ; et
la génération de secondes données de recalage en recalant les premières données de sous-recalage aux secondes données de sous-recalage.

2. Procédé selon la revendication 1, comprenant en outre l'obtention d'une image de segmentation de tomodensitométrie dans laquelle au moins l'un des éléments suivants : les dents maxillaires, les dents mandibulaires, l'os maxillaire, l'os mandibulaire et le canal du nerf mandibulaire, est segmenté par le processeur effectuant un traitement d'image sur l'image de tomodensitométrie.

3. Procédé selon la revendication 2,
dans lequel la génération des premières données de sous-recalage comprend :
la génération des premières données de sous-recalage en recalant l'image de numérisation de l'arcade maxillaire sur l'image de segmentation de tomodensitométrie, et
la génération des secondes données de sous-recalage comprend :
la génération des secondes données de sous-recalage en recalant l'image de numérisation de l'arcade mandibulaire sur l'image de segmentation de tomodensitométrie.

4. Procédé selon la revendication 3,
dans lequel la génération des premières données de recalage comprend :
le calcul d'une matrice de transformation des premières données de recalage aux secondes données de sous-recalage ; et
l'application d'une matrice de transformation inverse correspondant à la matrice de transformation à au moins l'un des éléments suivants : les dents mandibulaires segmentées, l'os mandibulaire segmenté et le canal nerveux mandibulaire segmenté.

5. Procédé selon la revendication 1,
dans lequel la génération des premières données de sous-recalage comprend :
la génération des premières données de sous-recalage à partir de l'image de numérisation de l'arcade maxillaire et de l'image de tomodensitométrie à l'aide d'un premier modèle d'apprentissage profond, et
le premier modèle d'apprentissage profond est un modèle d'intelligence artificielle construit en modélisant une corrélation entre des données d'entrée et des données de sortie selon un algorithme d'apprentissage profond, les données d'entrée comprenant une pluralité d'images de numérisation de l'arcade maxillaire et une pluralité d'ensembles d'images de tomodensitométrie provenant d'une pluralité de patients, et les données de sortie comprenant une pluralité d'ensembles de données de sous-recalage générées en recalant chacune des images de numérisation de l'arcade maxillaire avec chacune des images de tomodensitométrie.

6. Procédé selon la revendication 1,
dans lequel la génération des secondes données de sous-recalage comprend :
la génération des données de seconds sous-recalage à partir de l'image de numérisation de l'arcade mandibulaire et de l'image de tomodensitométrie à l'aide d'un second modèle d'apprentissage profond, et
le second modèle d'apprentissage profond est un modèle d'intelligence artificielle construit en modélisant une corrélation entre des données d'entrée et des données de sortie selon un algorithme d'apprentissage profond, les données d'entrée comprenant une pluralité d'images de numérisation de l'arcade mandibulaire et une pluralité d'ensembles d'images de tomodensitométrie provenant d'une pluralité de patients, et les données de sortie comprenant une pluralité d'ensembles de données de sous-recalage générées en recalant chacune des images de numérisation de l'arcade mandibulaire avec chacune des images de tomodensitométrie.

7. Procédé selon la revendication 1,
dans lequel les premières données de sous-recalage et les secondes données de sous-recalage sont générées à partir d'au moins trois repères issus des données tridimensionnelles du patient,
au moins trois repères sont extraits par une application permettant de générer des données de recalage dentaire,
les au moins trois repères sont extraits à l'aide d'un module de réseau neuronal artificiel intégré à l'application ou connecté via un réseau, et
le module de réseau neuronal artificiel est pré-entraîné à l'aide de données de pré-entraînement.

8. Dispositif destiné à générer des données de recalage dentaire, comprenant :
un circuit de communication ;
une mémoire ; et
un processeur,
dans lequel le processeur est configuré pour :
acquérir une image de numérisation intra-orale d'une région dentaire d'un patient, l'image de numérisation intra-orale comprenant une image de numérisation de l'arcade maxillaire et une image de numérisation de l'arcade mandibulaire,
obtenir une image de tomodensitométrie incluant la région dentaire du patient,
générer les premières données de sous-recalage en recalant l'image de numérisation de l'arcade maxillaire sur l'image de tomodensitométrie,
générer des premières données de recalage en recalant l'image de numérisation de l'arcade mandibulaire avec les premières données de sous-recalage,
générer des secondes données de sous-recalage en recalant l'image de numérisation de l'arcade mandibulaire sur l'image de tomodensitométrie, et
générer des secondes données de recalage en recalant les premières données de sous-recalage aux secondes données de sous-recalage.

9. Dispositif selon la revendication 8,
comprenant en outre :
un dispositif d'entrée ; et
un dispositif d'affichage,
dans lequel le processeur est configuré pour :
recevoir une entrée d'utilisateur par l'intermédiaire du dispositif d'entrée afin de sélectionner l'une des données de recalage parmi les premières données de recalage et les deuxièmes données de recalage, et
afficher les données de recalage sélectionnées sur le dispositif d'affichage en réponse à la réception de l'entrée d'utilisateur.

10. Dispositif selon la revendication 8, comprenant en outre :
un dispositif d'entrée ; et
un dispositif d'affichage,
dans lequel le processeur est configuré pour :
recevoir une entrée d'utilisateur par l'intermédiaire du dispositif d'entrée concernant un procédé de traitement de la région dentaire du patient,
sélectionner, parmi les premières données de recalage et les secondes données de recalage, celle qui corresponde à l'entrée d'utilisateur, et
afficher les données de recalage sélectionnés sur le dispositif d'affichage.

11. Dispositif selon la revendication 8,
dans lequel le processeur est configuré pour obtenir une image de segmentation de tomodensitométrie dans laquelle au moins l'un des éléments suivants : les dents maxillaires, les dents mandibulaires, l'os maxillaire, l'os mandibulaire et le canal du nerf mandibulaire, est segmenté par le biais d'un traitement d'image appliqué à l'image de tomodensitométrie.

12. Dispositif selon la revendication 11,
dans lequel le processeur est configuré pour :
générer les premières données de sous-recalage en recalant l'image de numérisation de l'arcade maxillaire sur l'image de segmentation de tomodensitométrie, et
générer les secondes données de sous-recalage en recalant l'image de numérisation de l'arcade mandibulaire sur l'image de segmentation de tomodensitométrie.

13. Dispositif selon la revendication 12,
dans lequel le processeur est configuré pour :
calculer une matrice de transformation des premières données de recalage aux secondes données de sous-recalage, et
appliquer une matrice de transformation inverse correspondant à la matrice de transformation à au moins l'un des éléments suivants : les dents mandibulaires segmentées, l'os mandibulaire segmenté et le canal nerveux mandibulaire segmenté.

14. Dispositif selon la revendication 8,
dans lequel le processeur est configuré pour générer les premières données de sous-recalage à partir de l'image de numérisation de l'arcade maxillaire et de l'image de tomodensitométrie à l'aide d'un premier modèle d'apprentissage profond,
le premier modèle d'apprentissage profond étant un modèle d'intelligence artificielle construit en modélisant une corrélation entre des données d'entrée et des données de sortie selon un algorithme d'apprentissage profond, les données d'entrée comprenant une pluralité d'images de numérisation de l'arcade maxillaire et une pluralité d'ensembles d'images de tomodensitométrie provenant d'une pluralité de patients, et les données de sortie comprenant une pluralité d'ensembles de données de sous-recalage générées en recalant chacune des images de numérisation de l'arcade maxillaire sur chacune des images de tomodensitométrie.

15. Dispositif selon la revendication 8,
dans lequel le processeur est configuré pour générer les secondes données de sous-recalage à partir de l'image de numérisation de l'arcade mandibulaire et de l'image de tomodensitométrie à l'aide d'un second modèle d'apprentissage profond,
le second modèle d'apprentissage profond étant un modèle d'intelligence artificielle construit en modélisant une corrélation entre des données d'entrée et des données de sortie selon un algorithme d'apprentissage profond, les données d'entrée comprenant une pluralité d'images de numérisation de l'arcade mandibulaire et une pluralité d'ensembles d'images de tomodensitométrie provenant d'une pluralité de patients, et les données de sortie comprenant une pluralité d'ensembles de données de sous-recalage générées en recalant chacune des images de numérisation de l'arcade mandibulaire avec chacune des images de tomodensitométrie.
